# EUROPEAN PATENT APPLICATION

(11) **EP 3 974 026 A1**
(43) Date of publication of application: **30.03.2022**
(21) Application number: 20809751.9
(22) Date of filing: 19.05.2020
(51) Int. Cl.: A61P 1/16, A23K 20/111, A23K 20/158, A23L 33/105, A23L 33/12, A61K 31/01, A61K 31/015

(54) **COMPOSITION FOR IMPROVING LIVER FUNCTION**

(30) Priority: 22.05.2019 JP 2019095658
(71) Applicant: Nitto Fuji Flour Milling Co., Ltd., Chuo-ku Tokyo 104-0033 (JP)
(72) Inventor: TAKAGI Youhei, Tokyo 1920982 (JP); HIRAGA Yumika, Tokyo 1920982 (JP); IMAI Shinjiro, Tokyo 1920982 (JP); KURASHINA Akie, Tokyo 143-0001 (JP); TAKAYANAGI Masayoshi, Tokyo 143-0001 (JP); SASAKI Yasuto, Tokyo 104-0033 (JP); OSHIMA Hideo, Tokyo 143-0001 (JP); KOIZUMI Takeshi, Tokyo 104-0033 (JP)
(74) Representative: Mewburn Ellis LLP
(86) International application number: PCT/JP2020/019742
(87) International publication number: WO 2020/235546

(57) **Abstract**

Provided is a composition for improving liver function that utilizes a plant-derived resorcinolic lipid, being useful, for example, in improving symptoms of non-alcoholic hepatitis.

A plant-derived resorcinolic lipid is used as an active ingredient of the composition for improving liver function. The resorcinolic lipid is preferably an alkylresorcinol and is also preferably derived from a gramineae plant, also preferably from wheat and/or rye bran and/or whole grains. This composition for improving liver function is especially suitably used to improve symptoms of non-alcoholic hepatitis.

### [Sequence listing]

SEQUENCE LISTING

<110>  NITTO FUJI FLOUR MILLING CO.,LTD.

<120>  Composition for improving liver function

<130>  200626WOP

<160>  14

<170>  PatentIn version 3.5

<210>  1
<211>  22
<212>  DNA
<213>  mouse

<220>
<221>  PPARgamma 5'-primer
<222>  (1)..(22)

<400>  1
tgtcggtttc agaagtgcct tg                                                22

<210>  2
<211>  24
<212>  DNA
<213>  mouse

<220>
<221>  PPARgamma 3'-primer
<222>  (1)..(24)

<400>  2
ttcagctggt cgatatcact ggag                                              24

<210>  3
<211>  20
<212>  DNA
<213>  mouse

<220>
<221>  Actin 5'-primer
<222>  (1)..(20)

<400>  3
tgacaggatg cagaaggaga                                                   20

<210>  4
<211>  20
<212>  DNA
<213>  mouse

<220>
<221>  Actin 3'-primer
<222>  (1)..(20)

<400>  4
gctggaaggt ggacagtgag                                                    20


<210>  5
<211>  23
<212>  DNA
<213>  mouse


<220>
<221>  PGC1alpha 5'-primer
<222>  (1)..(23)

<400>  5
aagtgtggaa ctctctggaa ctg                                                23


<210>  6
<211>  23
<212>  DNA
<213>  mouse


<220>
<221>  PGC1alpha 3'-primer
<222>  (1)..(23)

<400>  6
gggttatctt ggttggcttt atg                                                23


<210>  7
<211>  21
<212>  DNA
<213>  mouse


<220>
<221>  PPARalpha 5'-primer
<222>  (1)..(21)

<400>  7
aagtgcctgt ctgtcgggat g                                                  21


<210>  8
<211>  25
<212>  DNA
<213>  mouse


<220>
<221>  PPARalpha 3'-primer
<222>  (1)..(25)

<400>  8
ccagagattt gaggtctgca gtttc                                              25


<210>  9
<211>  23

<212>  DNA
<213>  mouse


<220>
<221>  Adiponectin 5′-primer
<222>  (1)..(23)

<400>  9
gtcagtggat ctgacgacac caa                                                23


<210>  10
<211>  19
<212>  DNA
<213>  mouse


<220>
<221>  Adiponectin 3′-primer
<222>  (1)..(19)

<400>  10
atgcctgcca tccaacctg                                                     19


<210>  11
<211>  19
<212>  DNA
<213>  mouse


<220>
<221>  mtDNA 5′-primer
<222>  (1)..(19)

<400>  11
aaccccgctc tacctcacc                                                     19


<210>  12
<211>  21
<212>  DNA
<213>  mouse


<220>
<221>  mtDNA 3′-primer
<222>  (1)..(21)

<400>  12
gtagcccatt tcttcccatt t                                                  21


<210>  13
<211>  20
<212>  DNA
<213>  mouse


<220>
<221>  genomeDNA 5′-primer
<222>  (1)..(20)

<400>  13
cgcggttcta ttttgttggt                                                    20

<210>  14
<211>  20
<212>  DNA
<213>  mouse

<220>
<221>  genomeDNA 3′-primer
<222>  (1)..(20)

<400>  14
agtcggcatc gtttatggtc                                                    20

## Description

### TECHNICAL FIELD

The present invention relates to a composition for improving liver function that utilizes a plant-derived component.

### BACKGROUND ART

Resorcinolic lipids are lipophilic substances having an alkyl group at the 2-position, 4-position, 5-position, and/or 6-position of the aromatic ring of resorcinol (1,3-dihydroxybenzene). In nature, large amounts are contained in the seeds of gramineae plants, especially the exodermis (bran), in ginkgo biloba, cashew nutshell oil, etc. In recent years, these resorcinolic lipids have been reported to possess various functionalities in humans, such as an oral antibacterial effect, immunosuppression, anti-diabetes effect, anti-inflammatory effect, intestinal flora regulation, anticholesterol effect, anti-obesity effect, sleep improvement, aging suppression, and circadian rhythm regulation (e.g., Patent Documents 1 to 10).

### [Related Art Documents]

### [Patent Documents]

[Patent Document 1] Japanese Patent No. 2613474
[Patent Document 2] Japanese Laid-Open Patent Application No. 2013-091612
[Patent Document 3] Japanese Laid-Open Patent Application No. 2014-139166
[Patent Document 4] Japanese Laid-Open Patent Application No. 2015-020993
[Patent Document 5] Japanese Laid-Open Patent Application No. 2015-218130
[Patent Document 6] Japanese Laid-Open Patent Application No. 2015-231986
[Patent Document 7] Japanese Laid-Open Patent Application No. 2016-132641
[Patent Document 8] Japanese Laid-Open Patent Application No. 2016-153387
[Patent Document 9] Japanese Patent No. 5926616
[Patent Document 10] Japanese Patent No. 5951448

### SUMMARY OF THE INVENTION

### [Problems the Invention is Intended to Solve]

However, there are no past reports of an effect that improves liver function in plant-derived resorcinolic lipids.

It is an objective of the present invention to provide a composition for improving liver function that utilizes a plant-derived resorcinolic lipid, being useful, for example, in improving symptoms of non-alcoholic hepatitis.

### [Means for Solving the Aforementioned Problems]

To achieve the above objective, the present invention, in a first aspect, provides a composition for improving liver function comprising a plant-derived resorcinolic lipid as an active ingredient.

In the composition for improving liver function according to the present invention, the resorcinolic lipid is preferably an alkylresorcinol.

Also, in the composition, the resorcinolic lipid is preferably derived from a gramineae plant.

Also, in the composition, the resorcinolic lipid is preferably derived from wheat and/or rye bran and/or whole grains.

Also, in the composition, the composition for improving liver function preferably comprises a solvent extract of a plant that contains the resorcinolic lipid.

Also, in the composition, when the composition for improving liver function comprises a solvent extract of a plant that contains the resorcinolic lipid, the solvent is preferably ethanol and/or hexane.

Also, in the composition, the composition for improving liver function is preferably used to improve symptoms of hepatitis.

Also, in the composition, the composition for improving liver function is preferably used to improve symptoms of non-alcoholic hepatitis.

Also, in the composition, the composition for improving liver function is preferably used to suppress triglyceride accumulation in the liver due to non-alcoholic hepatitis.

Also, in the composition, the composition for improving liver function is preferably provided as a food or beverage, a food additive, a pharmaceutical, a supplement, or an animal feed.

To achieve the above objective, the present invention, in the second aspect, provides a use of a plant-derived resorcinolic lipid to prepare a composition for improving liver function.

In the use of the plant-derived resorcinolic lipid according to the present invention, the resorcinolic lipid is preferably an alkylresorcinol.

Also, in the use, the resorcinolic lipid is preferably derived from a gramineae plant.

Also, in the use, the resorcinolic lipid is preferably derived from wheat and/or rye bran and/or whole grains.

Also, in the use, the composition for improving liver function preferably comprises a solvent extract of a plant that contains the resorcinolic lipid.

Also, in the use, when the composition for improving liver function comprises a solvent extract of a plant that contains the resorcinolic lipid, the solvent is preferably ethanol and/or hexane.

Also, in the use, the composition for improving liver function is preferably used to improve symptoms of hepatitis.

Also, in the use, the composition for improving liver function is preferably used to improve symptoms of non-alcoholic hepatitis.

Also, in the use, the composition for improving liver function is preferably used to suppress triglyceride accumulation in the liver due to non-alcoholic hepatitis.

Also, in the use, the composition for improving liver function is preferably provided as a food or beverage, a food additive, a pharmaceutical, a supplement, or an animal feed.

### [Effect of the Invention]

According to the present invention, a composition for improving liver function that utilizes a plant-derived resorcinolic lipid, being useful, for example, in improving symptoms of non-alcoholic hepatitis, can be provided.

### BRIEF DESCRIPTION OF THE DRAWINGS

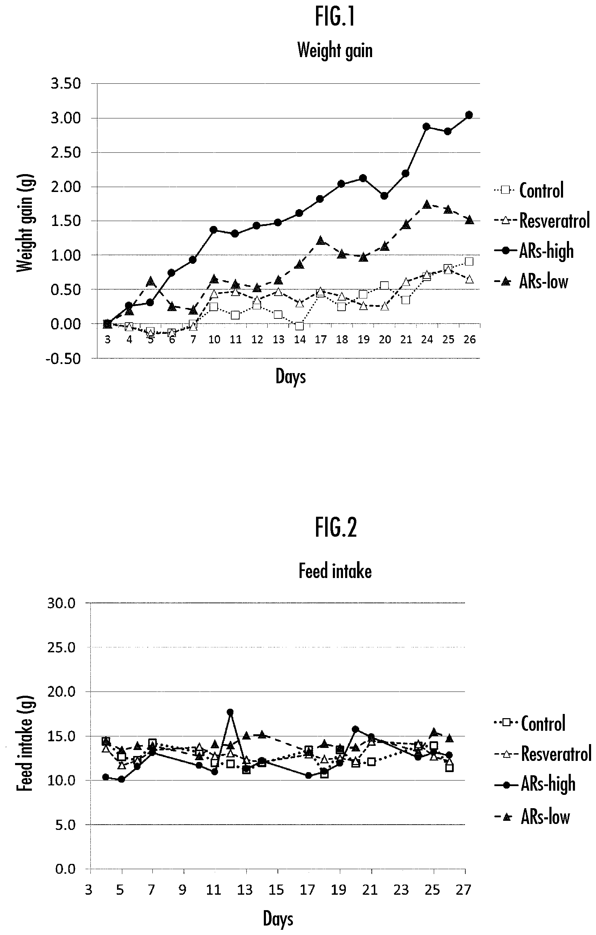

FIG. 1 is a chart showing the results of examining changes in weight gain in each group during the test period in Test Example 2.
FIG. 2 is a chart showing the results of examining changes in feed intake in each group during the test period in Test Example 2.

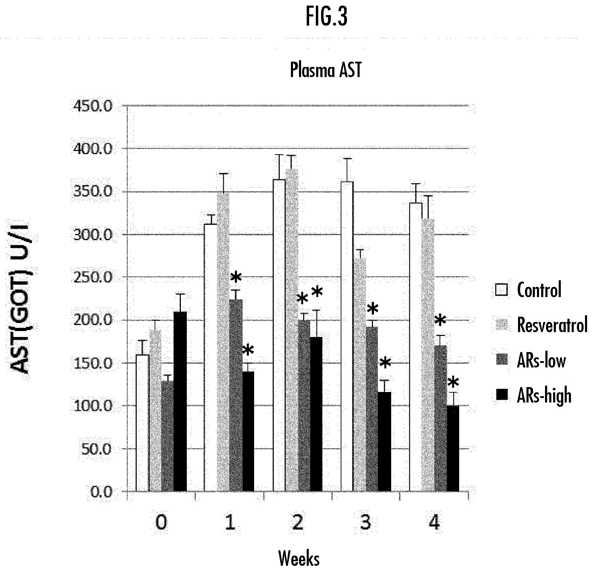

FIG. 3 is a chart showing the results of examining changes in AST values in each group during the test period in Test Example 3. The results were expressed by the mean and standard deviation in each group (n=5 to 6). Also, statistically significant differences from the control group (group C) were determined by Dunnett's multiple comparison test, and "*" in the chart indicates a significant difference at a risk of p<0.05.

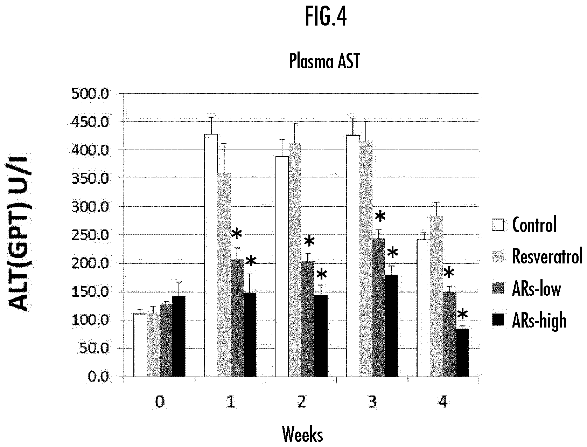

FIG. 4 is a chart showing the results of examining changes in ALT values in each group during the test period in Test Example 3. The results were expressed by the mean and standard deviation in each group (n=5 to 6). Also, statistically significant differences from the control group (group C) were determined by Dunnett's multiple comparison test, and "*" in the chart indicates a significant difference at a risk of p<0.05.

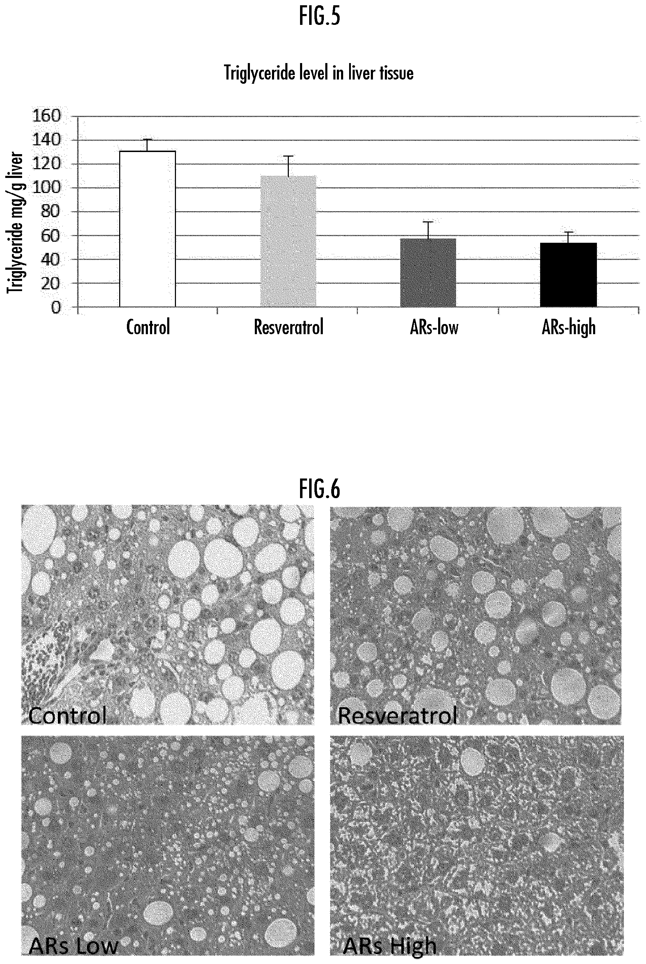

FIG. 5 is a chart showing the results of examining triglyceride levels in the liver tissue of each group in Test Example 4. The results were expressed by the mean and standard deviation in each group (n=5 to 6).
FIG. 6 is a chart showing the results of examining the liver tissue of each group by HE stain in Test Example 5.

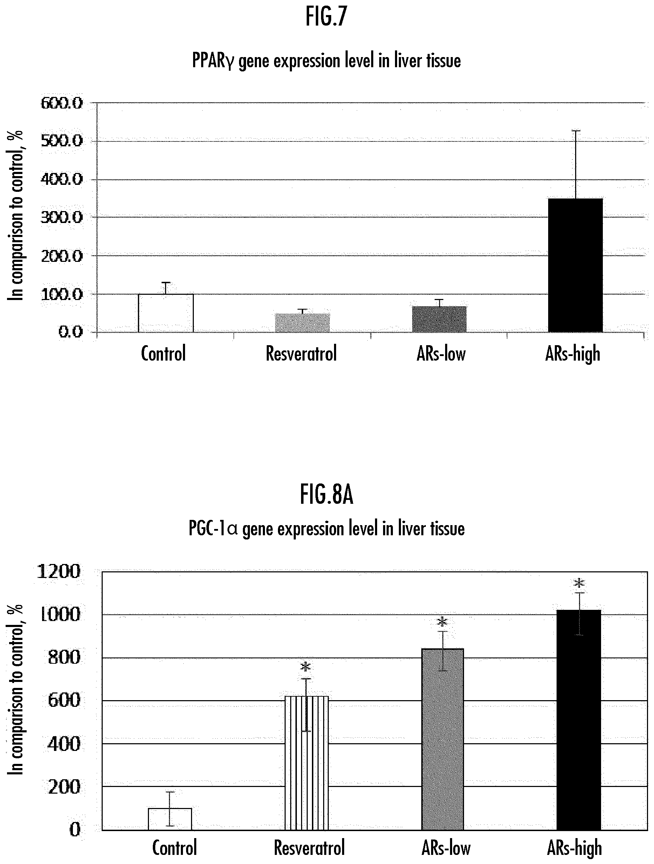

FIG. 7 is a chart showing the results of examining the PPARγ gene expression level in the liver tissue of each group in Test Example 6. The results were expressed by the mean and standard deviation in each group (n=5 to 6).
FIG. 8A is a chart showing the results of examining the PGC-1α gene expression level in the liver tissue of each group in Test Example 7. The results were expressed by the mean and standard deviation in each group (n=5 to 6). Also, statistically significant differences from the control group (group C) were determined by Dunnett's multiple comparison test, and "*" in the chart indicates a significant difference at a risk of p<0.05.

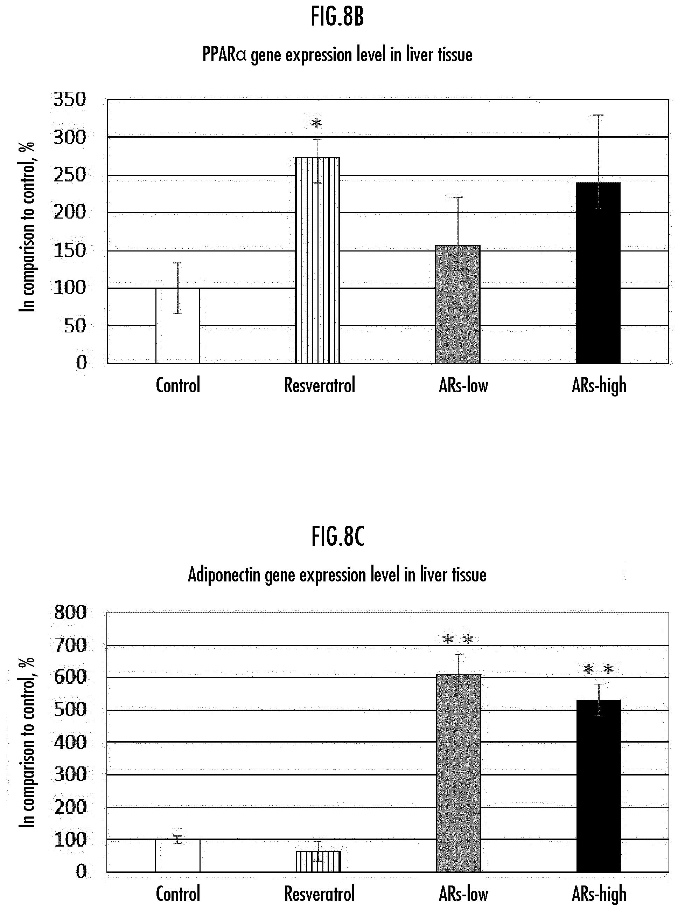

FIG. 8B is a chart showing the results of examining the PPARα gene expression level in the liver tissue of each group in Test Example 7. The results were expressed by the mean and standard deviation in each group (n=5 to 6). Also, statistically significant differences from the control group (group C) were determined by Dunnett's multiple comparison test, and "*" in the chart indicates a significant difference at a risk of p<0.05.
FIG. 8C is a chart showing the results of examining the adiponectin gene expression level in the liver tissue of each group in Test Example 7. The results were expressed by the mean and standard deviation in each group (n=5 to 6). Also, statistically significant differences from the control group (group C) were determined by Dunnett's multiple comparison test, and "*" in the chart indicates a significant difference at a risk of p<0.05.

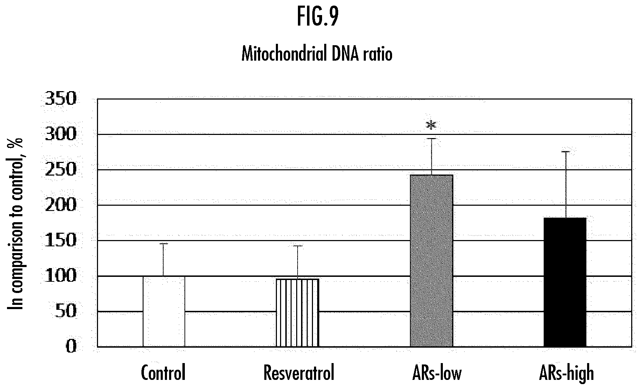

FIG. 9 is a chart showing the results of evaluating the intracellular mitochondria number in the liver tissue of each group by the weight ratio of mitochondrial DNA to genomic DNA in Test Example 8. The results were expressed by the mean and standard deviation in each group (n=5 to 6). Also, statistically significant differences from the control group (group C) were determined by Dunnett's multiple comparison test, and "*" in the chart indicates a significant difference at a risk of p<0.05.

### MODE FOR CARRYING OUT THE INVENTION

In the present invention, plant-derived resorcinolic lipids are used as an active ingredient of a composition for improving liver function.

As was mentioned above, resorcinolic lipids are lipophilic substances having an alkyl group at the 2-position, 4-position, 5-position, and/or 6-position of the aromatic ring of resorcinol (1,3-dihydroxybenzene). In nature, large amounts are seen in plants belonging to the families Anacardiaceae, Ginkgoaceae, Proteaceae, Myrsinoideae, Primulaceae, Myristicaceae, Iridaceae, Araceae, Fabaceae, Poaceae, etc., mugwort of the Asteraceae, etc. Since resorcinolic lipids are relatively abundant in the edible parts (seeds) of gramineae plants such as wheat and rye among these plants, these can be used suitably as a raw material for the resorcinolic lipids used in the present invention. For example, brans or whole grains of the edible parts (seeds) derived from wheat, rye, or the like contain about 0.015 to 0.3 mass% of alkylresorcinol compounds shown by formula (I). Furthermore, a fermented product such as miso made from wheat or rye can also be given as an example as a preferred raw material of the resorcinolic lipids used in the present invention.

Alkylresorcinol compounds represented by formula (I) are typical examples of the resorcinolic lipid used in the present invention.

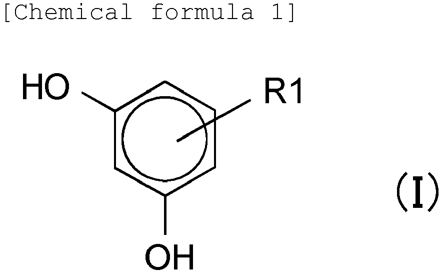

In formula (I), R1 represents a saturated or unsaturated linear or branched alkyl group. The carbon number of the alkyl group represented by R1 is not provided by way of limitation, and is preferably 1 to 27, more preferably 3 to 27, and even more preferably 5 to 27.

In formula (I), preferably, R1 is a saturated or unsaturated linear alkyl group, more preferably a saturated linear alkyl group. Examples of the saturated linear alkyl group represented by R1 include methyl, n-propyl, n-pentyl, n-heptyl, n-nonyl, n-undecyl, n-tridecyl, n-pentadecyl, n-heptadecyl, n-nonadecyl, n-heneicosyl, n-tricosyl, etc.

There are no particular limitations as to the position and number of unsaturated bonds in an unsaturated linear alkyl group represented by R1 in formula (I). Examples of the unsaturated linear alkyl group include alkyl groups having an unsaturated bond at any position on the carbon chain of the abovementioned saturated linear alkyl groups.

There are no particular limitations as to the position and number of branches or the position and number of unsaturated bonds of saturated or unsaturated branched alkyl groups represented by R1 in formula (I).

Preferred examples of compounds of formula (I) include the following.

5-Pentylresorcinol [olivetol, or 1,3-dihydroxy-5-n-pentylbenzene (C5:0)]
5-Heptylresorcinol [or 1,3-dihydroxy-5-n-heptylbenzene (C7:0)]
5-Nonylresorcinol [or 1,3-dihydroxy-5-n-nonylbenzene (C9:0)]
5-Undecylresorcinol [or 1,3-dihydroxy-5-n-undecylbenzene (C11:0)]
5-Tridecylresorcinol [or 1,3-dihydroxy-5-n-tridecylbenzene (C13:0)]
5-Pentadecylresorcinol [or 1,3-dihydroxy-5-n-pentadecylbenzene (C15:0)]
5-Heptadecylresorcinol [or 1,3-dihydroxy-5-n-heptadecylbenzene (C17:0)]
5-Nonadecylresorcinol [or 1,3-dihydroxy-5-n-nonadecylbenzene (C19:0)]
5-Heneicosylresorcinol [or 1,3-dihydroxy-5-n-heneicosylbenzene (C21:0)]
5-Tricosylresorcinol [or 1,3-dihydroxy-5-n-tricosylbenzene (C23:0)]
5-Pentacosylresorcinol [or 1,3-dihydroxy-5-n-pentacosylbenzene (C25:0)]
5-Heptacosylresorcinol [or 1,3-dihydroxy-5-n-heptacosylbenzene (C27:0)]

The plant-derived resorcinolic lipid used in the present invention may be, for example, the one obtained from commercial products or extracted from a plant by a usual method. For example, commercial products of compounds of formula (I) can be purchased from ReseaChem GmbH, Sigma-Aldrich, etc.

Examples of methods of extraction from plants include a method of immersing the raw material in a normal-temperature or heated extraction solvent under normal pressure or pressurization while stirring if necessary, reflux extraction methods, etc. The raw material may be cut, crushed, squeezed, dried, or a combination thereof prior to being added to the extraction solvent.

Examples of extraction solvents include alcohols that are liquid at room temperature such as methanol, ethanol, n-propanol, isopropanol, n-butanol, and other such lower alcohols, or 1,3-butylene glycol, propylene glycol, glycerin, or other such polyhydric alcohols; diethyl ether, propyl ether, and other such ethers; butyl acetate, ethyl acetate, and other such esters; acetone, ethyl methyl ketone, and other such ketones; hexane; and chloroform and other such organic solvents. These solvents may be used individually or in combinations of two or more. Among the above solvents, in terms of operability and effects on the environment, it is preferable to use alcohols that are liquid at room temperature, for example, lower alcohols having the carbon atom number of 1 to 4, more preferably ethanol from the viewpoint of safety due to residual solvent.

Hydrous organic solvents in which an aqueous component is contained in an organic solvent can also be included as extraction solvents. From the viewpoint of maintaining high extraction efficiency, the aqueous component content of the hydrous organic solvent is usually 50 vol% or less, preferably 30 vol% or less, and more preferably 20 vol% or less. Preferred examples of hydrous organic solvents include hydrous alcohols in which an aqueous component is contained in alcohols that are liquid at room temperature such as those mentioned above, more preferably hydrous ethanol.

Conditions for extraction such as temperature, time, etc. can be established as appropriate depending on the type of extraction solvent used, extraction conditions, etc., but an extraction temperature of from about 2°C to 100°C and extraction time of from about 30 minutes to 60 hours are preferred. The amount of extraction solvent is preferably from about 200 mass parts to 2000 mass parts in relative to 100 mass parts of raw material. Also, after extraction treatment, a mixture containing an infusion and a residue may be filtered, centrifuged, etc. as needed, and the solid component which is the residue may be removed. The solid component which has been removed may also be subjected to an extraction operation using an extraction solvent again, and this operation may be repeated several times. The extract obtained may be submitted to liquid chromatography, etc., and a further purified extract containing resorcinolic lipids may be prepared. In the case of such purification, the degree of purification in any form can be, for example, typically in a range of 1 mass% or higher as the plant-derived resorcinolic lipid content, more typically in a range of 5 mass% or higher, and even more typically in a range of 10 mass% or higher. Also, for example, the degree of purification may typically be in a range of 100 mass% or lower, more typically in a range of 90 mass% or lower, and even more typically in a range of 80 mass% or lower. The extract obtained can be used in the present invention in an unmodified state or after being subjected to further treatments such as concentration, freeze drying, hot-air drying, crushing, powdering, classification, dilution, mixing with water, etc. as needed.

As will be shown in the examples below, the plant-derived resorcinolic lipid has an effect of improving liver function. The present invention provides a composition for improving liver function based on this attribute of the resorcinolic lipid. Furthermore, the term "liver function" includes improvement of symptoms of hepatitis, for example, non-alcoholic hepatitis, alcoholic hepatitis, viral hepatitis, etc. Also, in addition to hepatitis, improvement of the symptoms of liver diseases such as non-alcoholic liver disease, fatty liver, liver cirrhosis, etc. can be mentioned. However, the application of a composition for improving liver function according to the present invention is not limited to these symptoms, and the composition can be used to improve a broad range of liver functions. Whether the symptoms have improved can be evaluated, for example, by measuring the aspartate aminotransferase (AST) value or alanine aminotransferase (ALT) value in the plasma, which are general indicators of liver function.

The dose of a composition for improving liver function according to the present invention can be varied as is appropriate in accordance with the type of individual to whom it is applied, symptoms, age, gender, etc. The dose in the case of a human subject can usually be 0.01 to 10 g per day in an adult of the abovementioned plant-derived resorcinolic lipid. The daily dose may be administered one time or divided over several times. Also, oral administration is more preferred. Furthermore, the subject of application is not limited to humans, and the composition can also be applied, for example, to dogs, cats, and other such animals.

There are no particular limitations as to the form of a composition for improving liver function according to the present invention, which may be any form that permits the abovementioned plant-derived resorcinolic lipid to be ingested. For example, the composition can assume the form of a solid, semi-solid, or liquid, or tablets, chewable tablets, powders, capsules, granules, drinks, gels, syrups, liquid foods for tube enteral feeding, etc. The composition may also assume the form of, for example, a health food, a functional food or beverage, a food or beverage for a specified health use, a food or beverage for the sick, etc., a food additive for the same, a pharmaceutical, a supplement, an animal feed for livestock, racehorses, exotic animals, pets, etc. Specifically, the form can be easily ingested by making the abovementioned plant-derived resorcinolic lipid part of each form. On the other hand, a composition for improving liver function according to the present invention may be constituted substantially of the abovementioned plant-derived resorcinolic lipid. Thus, the amount of the abovementioned plant-derived resorcinolic lipid in any form may be, for example, typically in a range of 0.1 mass% or higher, more typically in a range of 0.5 mass% or higher, and even more typically in a range of 1.0 mass% or higher. The amount may also be, for example, typically in a range of 100 mass% or lower, more typically in a range of 90 mass% or lower, and even more typically in a range of 80 mass% or lower.

Typical forms of pharmaceuticals include, for example, tablets, capsules, granules, powders, syrups, dry syrups, liquids, suspensions, and other such oral agents; inhalants; percutaneous preparations; suppositories and other such enteral preparations; and intravenous drip infusions, injections, and other such parenteral agents. The above liquids, suspensions, and other such liquid preparations may be in a form dissolved or suspended in water or another suitable vehicle immediately before use, and the above tablets and granules may have the surface coated by a known method. In addition, the above injections may be a solution or suspension of the abovementioned plant-derived resorcinolic lipid in a sterile distilled water or sterile saline, including a dissolution aid as needed.

Various pharmaceutically acceptable carriers, for example, excipients, stabilizers, other additives, etc., may be contained in the form of pharmaceuticals, or other medicinal ingredients, for example, various vitamins, minerals, crude drugs, etc., may be contained.

As typical forms of foods and beverages, green tea, oolong tea, black tea, and other such tea beverages, coffee beverages, soft drinks, jelly drinks, sports drinks, milk beverages, carbonated beverages, juice drinks, lactic acid bacteria beverages, fermented milk beverages, powdered drinks, cocoa beverages, alcoholic beverages, mineral water, and other such beverages; butter, jam, sprinkles, margarine, and other such spreads; mayonnaise; shortening; cream; dressings; breads; cooked rice; noodles; pasta; miso soup; tofu; cow's milk; yoghurt; soup or sauces; confections (e.g., biscuits or cookies, chocolates, candies, cakes, ice cream, chewing gum, tablets), etc. may be mentioned.

In the above form of foods and beverages, other food and beverage ingredients permitted in foods, various nutrients, various vitamins, minerals, amino acids, various fats and oils, various additives (e.g., taste components, sweeteners, organic acids, and other such acidulants, surfactants, pH regulators, stabilizers, antioxidants, colorings, flavors), etc. may be contained as needed, or other medicinal ingredients, for example, various vitamins, minerals, crude drugs, etc., may also be contained.

A typical form of food additive may be any composition or form that can be blended with foods and beverages such as those exemplified above.

A typical form of animal feed can be a composition or form basically the same as the composition or form of foods and beverages such as those exemplified above, except for animals.

A typical form of supplements is an oral composition among the forms mentioned above. For example, a supplement can be made in a form such as a tablet, chewable tablet, powder, capsule, granules, drink, gel, syrup, etc.

### [Examples]

The present invention is explained in greater detail below with reference to examples, but the present invention is not limited to the following examples.

### <Preparation example 1> Preparation of alkylresorcinol

Rye bran (approximately 9.4 kg) was dried under reduced pressure (80°C, 16 hours), a five-fold amount of 99% ethanol relative to the dried raw material was added, and stir extraction was performed under heating (60°C, one hour). Solid-liquid separation was performed thereafter to obtain a filtrate A. An equal amount of 99% ethanol was added to the residue, and solid-liquid separation was performed after rinsing to obtain a filtrate B. The filtrate A and filtrate B obtained were combined and concentrated under reduced pressure to obtain 516 g of a rye bran ethanol extract.

A quantity of 5.8 L of purified water and 5.8 L of 99% ethanol were added to 463 g of rye bran ethanol extract. After stirring for one hour on a 60°C water bath, 696 g of α-cyclodextrin was added, and stirring and mixing were performed for another hour. The product was spray-dried to obtain 649 g of a spray dried product. The particle size was also adjusted (10 mesh pass), and 639 g of rye bran ethanol extract dried product was obtained.

The dried product was dissolved in 2 L of hexane and purified by silica normal-phase chromatography. A Hi-Flash column silica 5 L catalog number: W007 (column inner diameter × column length: 60 × 180 mm, filler amount: 250 g) manufactured by Yamazen Co., Ltd. was used as the column. The chromatography conditions were a flow rate of 70 mL/min, detection wavelength of 280 nm, and mobile phase (hexane/ethyl acetate: step gradient of 90/10 (v/v) for nine minutes → 80/20 (v/v) for 15 minutes → 60/40 (v/v) for 16 minutes). Eluate with an elution time of from 30 minutes to 40 minutes was collected. The collected material was dried under reduced pressure, and 50 g of dry solid was obtained.

### <Test example 1> Analysis by high-performance liquid chromatography

The amount of alkylresorcinols in the dry solid obtained in Preparation example 1 was assayed via high-performance liquid chromatography analysis. Furthermore, alkylresorcinols are sometimes referred to as "ARs" or "AR" hereinafter.

Specifically, a GL Science Inertsil ODS4 was used as the analysis column, and analysis was performed using a flow rate of 1 mL/min, a detection wavelength of 275 nm, a column temperature of 40°C, and mobile phase (gradient of 89 v/v% methanol (5 minutes) → 92 v/v% methanol → (25 minutes) → 100% methanol) as analysis conditions. Commercial olivetol (AR having a C5 linear alkyl chain: Sigma-Aldrich) was used as the alkylresorcinol standard. For the quantification of each AR having an alkyl chain length different from that of olivetol, the elution position was specified in advance, and the measured peak area was calculated by multiplying by a coefficient, taking the value obtained by dividing the molecular weight of each by the molecular weight of olivetol as the coefficient.

As a result, the dry solid obtained in Preparation example 1 was confirmed to contain 6.8 g of ARs in 50 g.

### <Test example 2> Animal test by hepatitis model mice

Non-alcoholic steatohepatitis model mice (Japan Charles River Co., Ltd., C57BL/6NASH mice, male) were purchased at the age of six weeks, acclimated for two days, and reared six per cage. The mice were reared in a constant temperature/constant humidity room maintained on a 12-hour light-dark cycle (light: 8 a.m. to 8 p.m.). After the acclimation phase, the feeds shown below were fed ad libitum in accordance with the test group.

- Control group (group C): control feed not blended with test substance
- Resveratrol group (group R): feed blended with 0.4 mass% of resveratrol (Tokyo Chemical Industry Co., Ltd.)
- ARs high dose group (group AH): feed blended with 0.9 mass% of ARs
- ARs low dose group (group AL): feed blended with 0.3 mass% of ARs

The body weight was measured once a day during the test phase. Blood samples were also drawn once a week. Blood was drawn from the caudal vein of the mice, and plasma was obtained. The plasma was stored at -80°C until being used in measurement. Also, after the body weight was measured on the final day of the test, blood was drawn under anesthesia by diethyl ether. The liver was removed immediately after sacrifice, weighed, and frozen by dry ice. The liver was divided into halves, and each was placed in a 1.5 mL tube and frozen at -80°C.

FIG. 1 shows the changes in weight gain in each group during the test.

As shown in FIG. 1, virtually no weight gain was seen in the control group (group C) during the test phase. The mice used in this test example were six weeks old and would normally gain weight since they were growing mice. However, the fact that they were hepatitis model mice was thought to cause nutrient metabolic abnormalities and suppress weight gain. In contrast, weight gain was confirmed in the ARs low dose group (group AL) and ARs high dose group (group AH). This was thought to be because ARs intake suppressed the hepatitis and, as a result, also improved the nutrient metabolic abnormalities. On the other hand, weight gain suppression in the hepatitis model mice could not be improved in the group that ingested resveratrol (group R), which is known to have the effect of suppressing fat accumulation in organs such as the liver. This was thought to be because the effect of ARs is not simply an effect resulting from improvement of general fat metabolism but a unique effect on the symptoms of hepatitis.

On the other hand, FIG. 2 shows the changes in feed intake in each group during the test phase. As shown in FIG. 2, no major difference in feed intake could be seen in any group.

### <Test example 3> Measurement of AST value and ALT value in plasma

The aspartate aminotransferase (AST) value and alanine aminotransferase (ALT) value in the plasma, which are general indicators of liver function, were measured. Measurement was conducted using a Transaminase CII-Test Wako (manufactured by Fuji Film Wako Pure Chemicals Co., Ltd.) according to the company's instructions.

FIG. 3 shows the changes in the AST values in each group during the test phase.

As shown in FIG. 3, the AST value rose from week 0 to week 4 in the control group (group C), and advance of hepatitis was confirmed. In contrast, increases in the AST values were suppressed in both the ARs low dose group (group AL) and the ARs high dose group (group AH). The degree of suppression was more pronounced in the high dose group than the low dose group. On the other hand, the changes were almost the same as the control group (group C) in the resveratrol group (group R).

FIG. 4 shows the changes in the ALT values in each group during the test phase.

As shown in FIG. 4, the ALT value rose from week 0 to week 4 in the control group (group C), and advance of hepatitis was confirmed. In contrast, increases in the ALT value were suppressed in both the ARs low dose group (group AL) and the ARs high dose group (group AH). The degree of suppression was more pronounced in the high dose group than the low dose group. On the other hand, the changes were almost the same as the control group (group C) in the resveratrol group (group R).

As shown in the results of FIGS. 3 and 4 above, it was clarified that ARs have the effect of suppressing hepatitis in an intake-amount-dependent manner. This effect was a unique effect on the symptoms of hepatitis that was not achieved by resveratrol, which is generally known to suppress fat accumulation in organs such as the liver.

### <Test example 4> Measurement of triglyceride level in liver tissue

Fifty milligrams of frozen liver tissue was placed in a tube, and 1 mL of a mixed solution containing chloroform and methanol in a 2:1 liquid volume ratio was added to the tissue. The tissue was crushed to a paste by a homogenizer, and the entire mixture was agitated for 15-20 minutes at room temperature using a shaker. The homogenate was centrifuged for five minutes at 15,000 rpm, and the supernatant was recovered. A quantity of 0.5 mL of normal saline was added to the recovered supernatant and mixed for several seconds. The mixture was centrifuged for one minute at 2000 rpm, and an operation to remove the upper phase was repeated twice. The lower chloroform phase was dried under reduced pressure. The dry solid was suspended in amounts of 100 µL each by ethanol, and 45 µL each was added to ethanol and mixed. The resultant mixture served as a sample, and the triglyceride level was measured.

Measurement was conducted using a triglyceride E-Test Wako (manufactured by Fuji Film Wako Pure Chemicals Co., Ltd.) according to the company's instructions. The results are shown in FIG. 5.

As shown in FIG. 5, the triglyceride level in the control group (group C) was 130 mg/g, showing a clear fatty liver value. In contrast, the value was halved to 60 mg/g or lower in both the ARs low dose group (group AL) and ARs high dose group (group AH). These values fell within the normal triglyceride level range. On the other hand, the triglyceride level in the liver tissue in hepatitis model mice could not be corrected in the group that ingested resveratrol (group R), which is known to have the effect of suppressing fat accumulation in organs such as the liver. Thus, the effect of alkylresorcinol intake was thought to be not simply an effect resulting from improvement of general fat metabolism but a unique effect on the symptoms of hepatitis.

### <Test example 5> Pathology evaluation of liver tissue

Frozen liver tissue was embedded in paraffin and sliced. The slices prepared were hematoxylin-eosin (HE) stained by the usual method. The results of HE staining of the liver tissue of each group are shown in FIG. 6.

As shown in FIG. 6, blank spaces derived from fat droplets were confirmed in the liver tissue of the control group (group C). These blank spaces were large and numerous, showing a clear image of fatty liver. In contrast, the number and size of the blank spaces were suppressed in both the ARs low dose group (group AL) and ARs high dose group (group AH). On the other hand, virtually no effect was seen in the group that ingested resveratrol (group R). Thus, in the same way as the results of Test example 4, the effect of correcting the triglyceride level in liver tissue by alkylresorcinol intake was thought to be not simply an effect resulting from improvement of general fat metabolism but a unique effect on the symptoms of hepatitis.

### <Test example 6> Measurement of PPARγ gene expression level in liver tissue

Peroxisome proliferator-activated receptor γ (PPARγ) is a protein belonging to the nuclear receptor superfamily and is known to play an important role in lipid metabolism. Pioglitazone (used as a diabetes drug), which is a ligand of this PPARγ, is also effective as a nonalcoholic hepatitis therapeutic. It was therefore thought that enhancing PPARγ function would be a useful target for non-alcoholic hepatitis treatment. Therefore, the expression level of the PPARγ gene in liver tissue was examined in this test example. Specifically, the expression level was measured by quantitative PCR as follows.

Total RNA was prepared from liver tissue using a "NucleoSpin (registered trademark) RNA II kit (manufactured by Takara Bio Inc.)" according to the company's instructions. cDNA was synthesized from total RNA using a "Protoscript (registered trademark) II First strand cDNA Synthesis Kit (manufactured by New England BioLabs Inc.)" according to the company's instructions.

Quantitative PCR using the prepared cDNA was performed using a "MyGo Mini Real Time PCR device (manufactured by Funakoshi Co., Ltd.)" and Luna universal LPCR Mix (manufactured by New England BioLabs Inc.) according to the companies' instructions. Primers having the following sequences were used.

(For PPARγ)
   Forward: 5'-TGTCGGTTTCAGAAGTGCCTTG-3' (SEQ ID NO: 1)
   Reverse: 5'-TTCAGCTGGTCGATATCACTGGAG-3' (SEQ ID NO: 2)
(For β-actin)
   Forward: 5'-TGACAGGATGCAGAAGGAGA-3' (SEQ ID NO: 3)
   Reverse: 5'-GCTGGAAGGTGGACAGTGAG-3' (SEQ ID NO: 4)

The PCR reaction was carried out under the following conditions.
(95°C, 30 seconds → 95°C, 5 seconds → 60°C, 30 seconds) × 40 cycles → 95°C, 60 seconds

For the peroxisome proliferator-activated receptor γ (PPARγ) gene expression level, the relative values of each group were compared, taking the control group (group C) as 100%. The results are shown in FIG. 7.

As shown in FIG. 7, in the ARs high dose group (AH), the PPARγ gene expression level increased about 3.5-fold relative to the control group (group C) in the liver tissue. Thus, an effect that enhances the expression of the PPARγ gene was suggested to participate in the liver function improving effect by alkylresorcinols.

### <Test example 7> Measurement of expression levels of various genes in liver tissue

The gene expression levels in liver tissue of various genes known to play important roles in lipid metabolism [peroxisome proliferator-activated receptor γ coactivator-la (PGC-1α), peroxisome proliferator-activated receptor PPARα, and adiponectin] were examined. Measurement was conducted by quantitative PCR in the same way as in Test example 6. Primers having the following sequences were used for this purpose.

(For PGC-1α)
   Forward: 5'-AAGTGTGGAACTCTCTGGAACTG-3' (SEQ ID NO: 5)
   Reverse: 5'-GGGTTATCTTGGTTGGCTTTATG-3' (SEQ ID NO: 6)
(For PPARα)
   Forward: 5'-AAGTGCCTGTCTGTCGGGATG-3' (SEQ ID NO: 7)
   Reverse: 5'-CCAGAGATTTGAGGTCTGCAGTTTC-3' (SEQ ID NO: 8)
(For adiponectin)
   Forward: 5'-GTCAGTGGATCTGACGACACCAA-3' (SEQ ID NO: 9)
   Reverse: 5'-ATGCCTGCCATCCAACCTG-3' (SEQ ID NO: 10)
(For β-actin)
   Forward: 5'-TGACAGGATGCAGAAGGAGA-3' (SEQ ID NO: 3)
   Reverse: 5'-GCTGGAAGGTGGACAGTGAG-3' (SEQ ID NO: 4)

The PCR reaction was carried out under the following conditions.
(95°C, 30 seconds → 95°C, 5 seconds → 60°C, 30 seconds) × 40 cycles → 95°C, 60 seconds

For the gene expression levels in the liver tissue, the relative values of each group were compared, taking the control group (group C) as 100%. The results are shown in FIGS. 8A-8C.

As shown in FIGS. 8A to 8C, the expression levels of the PGC-1α, PPARα, and adiponectin genes in liver tissue increased markedly in the ARs groups. In particular, the PGC-1α and adiponectin gene expression levels were even increased in comparison to the positive control resveratrol group (group R) (FIGS. 8A, C). Thus, an effect that enhances expression of these genes was suggested to participate in the liver function improving effect by alkylresorcinols.

### <Test example 8> Evaluation of intracellular mitochondria number of liver tissue

Enhancement of the gene expression of PGC-1α, the gene expression level of which was confirmed to be increased in the alkylresorcinol groups in Test example 7, is known to raise the mitochondria number, promote oxidative phosphorylation, activate the TCA cycle, promote lipid combustion by β-oxidation, cause gluconeogenesis, promote ketone body synthesis, etc.

In this test example, the intracellular mitochondria number of the liver tissue was examined. Specifically, the intracellular mitochondria number of the liver tissue was evaluated by comparing the weight ratio of mitochondrial DNA to genomic DNA, measured by quantitative PCR as follows.

DNA was extracted from liver tissue using a "NucleoSpin (registered trademark) Tissue kit (manufactured by Takara Bio Inc.)" according to the company's instructions. Each DNA copy number was quantified using a "Mouse Feeder Cell Quantification Kit (manufactured by Takara Bio Inc.)" according to the company's instructions. Primers having the following sequences were used.

(For mitochondrial DNA)
   Forward: 5'-AACCCCGCTCTACCTCACC-3' (SEQ ID NO: 11)
   Reverse: 5'-GTAGCCCATTTCTTCCCATTT-3' (SEQ ID NO: 12)
(For genomic DNA)
   Forward: 5'-CGCGGTTCTATTTTGTTGGT-3' (SEQ ID NO: 13)
   Reverse: 5'-AGTCGGCATCGTTTATGGTC-3' (SEQ ID NO: 14)

The PCR reaction was carried out under the following conditions.
(95°C, 30 seconds → 95°C, 5 seconds → 60°C, 30 seconds) × 40 cycles → 95°C, 60 seconds

For the weight ratio of measured mitochondrial DNA to genomic DNA, the relative values of each group were compared, taking the control group (group C) as 100%. The results are shown in FIG. 9.

As shown in FIG. 9, in the resveratrol group (group R), no changes in intracellular mitochondria number were seen in comparison to the control group (group C). In contrast, in the ARs low dose group (group AL) and ARs high dose group (group AH), the intracellular mitochondria number increased. Thus, an effect of increasing the liver tissue intracellular mitochondria number was suggested to participate in the liver function improving effect by alkylresorcinol intake.

## Claims

1. A composition for improving liver function comprising a plant-derived resorcinolic lipid as an active ingredient.

2. The composition for improving liver function according to Claim 1, wherein the resorcinolic lipid is an alkylresorcinol.

3. The composition for improving liver function according to Claims 1 or 2, wherein the resorcinolic lipid is derived from a gramineae plant.

4. The composition for improving liver function according to any of Claims 1 to 3, wherein the resorcinolic lipid is derived from wheat and/or rye bran and/or whole grains.

5. The composition for improving liver function according to any of Claims 1 to 4, comprising a solvent extract of a plant that contains the resorcinolic lipid.

6. The composition for improving liver function according to Claim 5, wherein the solvent is ethanol and/or hexane.

7. The composition for improving liver function according to any of Claims 1 to 6, to be used to improve symptoms of hepatitis.

8. The composition for improving liver function according to Claim 7, to be used to improve symptoms of non-alcoholic hepatitis.

9. The composition for improving liver function according to Claim 8, to be used to suppress triglyceride accumulation in the liver due to non-alcoholic hepatitis.

10. The composition for improving liver function according to any of Claims 1 to 9, provided as a food or beverage, a food additive, a pharmaceutical, a supplement, or an animal feed.

11. A use of a plant-derived resorcinolic lipid to prepare a composition for improving liver function.

12. The use according to Claim 11, wherein the resorcinolic lipid is an alkylresorcinol.

13. The use according to Claims 11 or 12, wherein the resorcinolic lipid is derived from a gramineae plant.

14. The use according to any of Claims 11 to 13, wherein the resorcinolic lipid is derived from wheat and/or rye bran and/or whole grains.

15. The use according to any of Claims 11 to 14, wherein the composition for improving liver function comprises a solvent extract of a plant that contains the resorcinolic lipid.

16. The use according to Claim 15, wherein the solvent is ethanol and/or hexane.

17. The use according to any of Claims 11 to 16, wherein the composition for improving liver function is used to improve symptoms of hepatitis.

18. The use according to Claim 17, wherein the composition for improving liver function is used to improve symptoms of non-alcoholic hepatitis.

19. The use according to Claim 18, wherein the composition for improving liver function is used to suppress triglyceride accumulation in the liver due to non-alcoholic hepatitis.

20. The use according to any of Claims 11 to 19, wherein the composition for improving liver function is provided as a food or beverage, a food additive, a pharmaceutical, a supplement, or an animal feed.
